# EUROPÄISCHE PATENTSCHRIFT

(11) **EP 1 448 260 B1**
(45) Veröffentlichungstag und Bekanntmachung des Hinweises auf die Patenterteilung: **27.04.2005**
(21) Anmeldenummer: 02798246.1
(22) Anmeldetag: 20.11.2002
(51) Int. Cl.: A61M 25/06

(54) **ABDECKGEHÄUSE EINER FLÜGELKANÜLE**
PROTECTIVE HOUSING FOR A BUTTERFLY NEEDLE
BOITIER DE PROTECTION D'UNE AIGUILLE A AILETTES

(30) Priorität: 20.11.2001 DE 10156587
(43) Veröffentlichungstag der Anmeldung: 25.08.2004
(73) Patentinhaber: Sarstedt AG & Co., 51588 Nümbrecht (DE)
(72) Erfinder: SARSTEDT, Walter, 51588 Nümbrecht (DE)
(74) Vertreter: Valentin, Ekkehard, Dipl.-Ing.
(86) Internationale Anmeldenummer: PCT/DE2002/004267
(87) Internationale Veröffentlichungsnummer: WO 2003/045491

(56) Entgegenhaltungen:
- EP-A- 0 499 077
- EP-B- 0 436 646
- WO-A-97/25082
- US-A- 5 827 239
- US-A- 5 921 969

## Beschreibung

Die Erfindung betrifft ein Abdeckgehäuse einer Flügelkanüle, bestehend aus einer im Querschnitt im wesentlichen jeweils U-förmigen langgestreckten Unterkappe und einer langgestreckten Oberkappe, die miteinander verbunden und am gegenüberliegenden Ende nach dem Einfügen der Flügel der Flügelkanüle in von den Kappen in Gehäuselängsrichtung bereitgestellten, mit einer vorderen und einer hinteren Halte- bzw. Verriegelungsposition ausgebildeten beidseitigen Führungsnuten zu einer Gehäuseeinheit verschließbar sind.

Zur Blutentnahme wird eine Kanüle, die über einen Verbindungsschlauch und einen Adapter an ein Blutentnahmeröhrchen angekoppelt werden kann, verwendet, die seitliche Flügel aufweist. Um zu verhindern, daß der Benutzer einer solchen Kanüle sich unbeabsichtigt an der Kanüle verletzt, werden solche Flügelkanülen mit einem Abdeckgehäuse versehen, welches die Kanüle einhüllt.

Ein solches Abdeckgehäuse ist durch die EP 0 436 646 B1 bekanntgeworden. Die Kanüle wird bei dieser Ausführung durch den mit der Kanüle verbundenen Schlauch aus dem Patienten gezogen. Um den Benutzer vor einem ungewollten Nadelstich zu schützen, wird die zurück- und eingezogene Nadel in einer geschützten Position arretiert. Zur Fixierung der Schutzvorrichtung wird ein Ankerteil gegen den Patienten gedrückt. Durch diese Handhabung wird das Risiko eines versehentlichen Stechens an der Nadel für den Benutzer minimiert bzw. vermieden.

Bei dieser bekannten Ausführung wird die Flügelkanüle direkt aus der Haut des Patienten in die geschützte Position des Abdeckgehäuses gezogen.

Diese Handhabung belastet den Patienten, wenn der Anker auf die Haut des Patienten gedrückt und gleichzeitig mit der anderen Hand an dem Schlauch gezogen wird. Es besteht die Möglichkeit, daß die Kanüle, die sich zu diesem Zeitpunkt in dem Blutgefäß des Patienten befindet, bewegt wird bzw. eine Bewegung ausführen möchte. Diese Bewegung erzeugt aber Schmerzen.

Der Erfindung liegt die Aufgabe zugrunde, ein Abdeckgehäuse der eingangs genannten Art zur einfacheren Handhabung so weiterzubilden, daß beim Zurückziehen der Flügelkanüle keine Widerstände überwunden werden müssen und das Zurückziehen der Kanüle in die Dauerverriegelungsposition außerhalb der Vene vorgenommen werden kann.

Diese Aufgabe wird mit den im kennzeichnenden Teil des unabhängigen Anspruchs angegebenen Merkmalen gelöst.

Nach einer Ausführung kann hierbei die Oberkappe schmaler sein als die Unterkappe, wobei die Führungsnuten von vertikalen und horizontalen beidseitigen freien Spaltabständen zwischen den zueinander benachbarten vertikalen Seitenwänden der Kappen gebildet werden. Von zumindest einer Seitenwand der Kappe ragt mindestens ein Federrastmittel in die Führungsnut und arretiert die flügelkanüle in der hinteren Dauerverriegelungsposition.

Nach einer anderen Ausführung können Ober- und Unterkappe deckungsgleich sein. In den vertikalen Spalt von durchgängig linear ausgebildeten Führungsnuten ragt Von zumindest einer Seitenwand der Kappe her ein der Dauerverriegelungsposition vorgeschaltetes Federrastmittel in die Führungsnut hinein.

Die vordere Halteposition der Flügelkanüle läßt sich vorzugsweise dadurch sichern, daß von dem Profilgrund der Oberkappe oder der Unterkappe oder von beiden Profilgründen Haltemittel vorspringen, die in Kontakt mit dem Mittelteil der Flügelkanüle sind. Diese Haltemittel können z.B. als Nocken oder als Stege bzw. Steg ausgebildet sein.

Weitere Merkmale und Vorteile der Erfindung ergeben sich aus den Ansprüchen und der nachfolgenden Beschreibung eines in den Zeichnungen dargestellten Ausführungsbeispiels der Erfindung. Es zeigen:
- Fig. 1: in perspektivischer Seitenansicht eine Blutentnahmevorrichtung, bestehend aus einer Flügelkanüle mit einem Abdeckgehäuse;

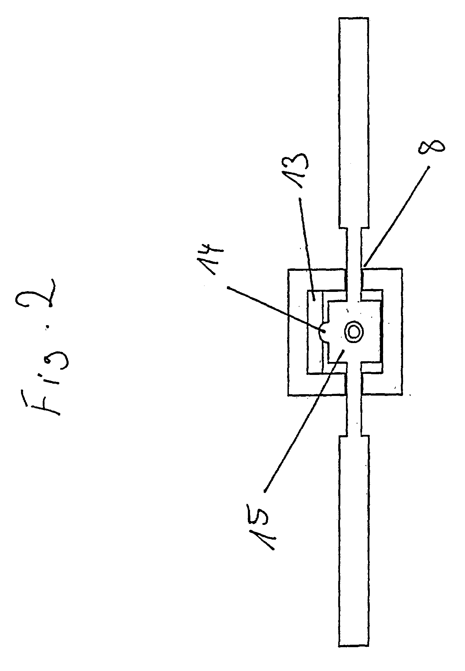

- Fig. 2: einen Schnitt entlang der Linie X-X aus Fig. 1; und

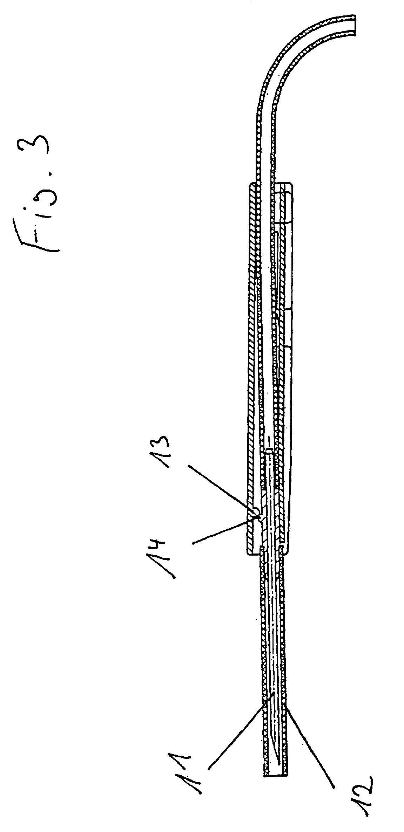

- Fig. 3: einen Längsschnitt der Blutentnahmevorrichtung nach Fig. 1

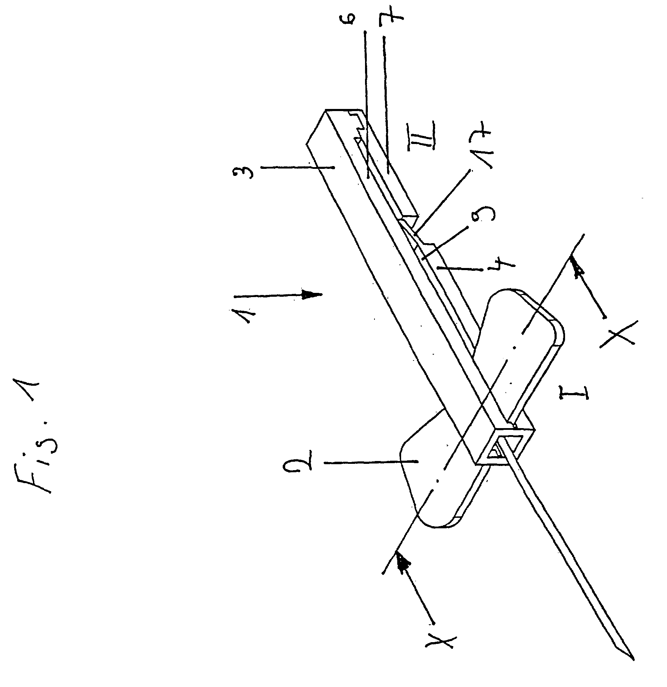

Von einer hinlänglich bekannten Blutentnahmevorrichtung einschließlich eingesetzter Flügelkanüle ist in Fig. 1 ein die Flügelkanüle 2 aufnehmendes Abdeckgehäuse 1 gezeigt. Das Abdeckgehäuse besteht aus einer Oberkappe 3 und einer Unterkappe 4. In der gezeigten Ausführung sind die Oberkappe 3 und die Unterkappe 4 gleich breit. Es ist aber auch möglich, daß die Oberkappe 3 schmaler als die Unterkappe 4 ist.

Die Oberkappe 3 kann mit der Unterkappe 4 über z. B. ein Filmscharnier verbunden sein. Nach dem Einlegen der Flügelkanüle 2 werden die Oberkappe 3 und die Unterkappe 4 durch z.B. eine Hakenverbindung verrastet.

Eine weitere Möglichkeit besteht darin, daß die Oberkappe 3 und die Unterkappe 4 beidseitig verrastet werden. Die jeweils mit einem vertikalen Abstand von einander benachbarten Seitenwände 6 bzw. 7 der Oberkappe 3 bzw. Unterkappe 4 ermöglichen einen horizontalen Spalt 8, der Führungsnuten 9 für die Flügel der Kanüle bereitstellt.

Ist die Oberkappe 3 schmaler als die Unterkappe 4, werden ein horizontaler Spalt 8 und ein vertikaler Spalt gebildet, durch den die Flügel der Kanüle 2 hindurchgleiten.

Befindet sich die Flügelkanüle 2 in der Ausgangsposition, d.h. in der Position, in der die Vorrichtung beim Benutzer ankommt, so ist die Kanüle 11 durch einen Schutzschlauch 12 abgedeckt. Ein elastischer Steg oder Nocken 13 bewirkt eine Arretierung der Flügelkanüle 2 dadurch, daß er mit einer Erhöhung 14 auf dem Mittelteil 15 der Flügelkanüle 2 in Wirkverbindung steht.

Nach der Blutentnahme wird die Flügelkanüle 2 aus der Vene des Patienten gezogen. Beim Zurückziehen der Flügelkanüle 2 wird die Erhöhung 14 auf dem Mittelteil 15 der Flügelkanüle 2 unter dem elastischen Steg oder Nocken 13 hindurchgezogen. Am Ende der Führungsnuten 9 werden die Flügel in einer Dauerverriegelungsposition II durch in die Führungsnuten 9 hineinragende Federrastmittel 17 gesichert. Die Federrastmittel 17 werden von den Flügeln ohne Widerstand zu leisten niedergedrückt.

## Patentansprüche

1. Abdeckgehäuse (1) einer Flügelkanüle (2), bestehend aus einer im Querschnitt im wesentlichen jeweils U-förmigen langgestreckten Unterkappe (4) und einer langgestreckten Oberkappe (3), die nach dem Einfügen der Flügel der Flügelkanüle (2) zu einer Gehäuseeinheit verschließbar sind,
**dadurch gekennzeichnet,**
**daß** Führungsnuten (9) für die Flügel von an beiden Gehäuseseiten freien Spaltabständen zwischen den zueinander benachbarten vertikalen Seitenwänden (6, 7) der Kappen (3, 4) gebildet sind und ein einer Dauerverriegelungsposition (II) zur Festlegung der Flügelkanüle (2) vorgeschaltetes Federrastmittel (17) von zumindest einer der Seitenwände (6, 7) in eine der Führungen (9) hineinragt.

2. Abdeckgehäuse nach Anspruch 1,
**dadurch gekennzeichnet,**
**daß** eine vordere Halteposition (I) der Flügelkanüle (2) mindestens eine vom Profilgrund der Kappen (3, 4) vorspringende Erhöhung (13) aufweist, die mit einer Erhöhung (14) auf dem Mitteteil der Flügelkanüle (2) in Wirkverbindung steht.

## Claims

1. Cover housing (1) of a wing cannula (2), comprising a longitudinally extending lower cap (4) having a substantially U-shaped cross section and a longitudinally extending upper cap (3), which are connectable to a housing unit after insertion of the wings of the wing cannula,
**characterized in that**,
the guide grooves (9) for the wings are formed by gap spacings free from both housing sides between adjacent, vertical side walls (6, 7) of the caps (3, 4) and a spring catch means (17) upstream of a permanent locking position (II) for fixing the wing cannula (2) from at least one of the side walls (6, 7) extends into one of the guides (9).

2. Cover housing according to claim 1, **characterized in that** a front holding position (I) of the wing cannula (2) has at least one ridge (13) projecting from the profile base of the caps (3, 4), which is connected with a ridge (14) on the center part of the wing cannula (2).

## Revendications

1. Boîtier de recouvrement (1) d'une canule à ailettes (2), constitué d'un capuchon inférieur allongé, respectivement sensiblement en forme de U dans sa section transversale et d'un capuchon supérieur allongé (3), qui après insertion des ailettes de la canule à ailettes (2) peut être fermé en une unité de boîtier,
**caractérisé en ce que**
des rainures de guidage (9) pour les ailettes sont formées par des intervalles libres entre les deux parois latérales verticales (6, 7) voisines entre elles des capuchons (3, 4) et un moyen d'enclenchement à ressort (17) monté en amont d'une position de verrouillage permanent (II) pour la fixation de la canule à ailettes (2) saillit à partir d'au moins l'une des parois latérales (6, 7) dans l'un des guidages (9).

2. Boîtier de recouvrement selon la revendication 1, **caractérisé en ce qu'**une position d'arrêt antérieure (I) de la canule à ailettes (2) présente au moins une élévation (13) saillant à partir du fond du profil des capuchons (3, 4) qui est en interaction avec une élévation (14) sur la partie médiane de la canule à ailettes (2).
